(19) Europäisches Patentamt
European Patent Office
Office européen des brevets

(11) Veröffentlichungsnummer: **0 018 360**
**B1**

(12) **EUROPÄISCHE PATENTSCHRIFT**

(45) Veröffentlichungstag der Patentschrift: **30.09.81**

(21) Anmeldenummer: **78900312.6**

(22) Anmeldetag: **16.12.78**

(86) Internationale Anmeldenummer: **PCT/DE78/00040**

(87) Internationale Veröffentlichungsnummer: **WO 79/00426 12.07.79 Gazette 79/15**

(51) Int. Cl.³: **C 07 D 405/06,**
A 61 K 31/495 //A61K31/34,
(C07D405/06, 307/83,
241/04)

# (54) N-(5-Methoxybentofuran-2-ylcarbonyl)-N'-benzylpiperazin und Verfahren zu dessen Herstellung.

(30) Priorität: **23.12.77 DE 2757532**

(43) Veröffentlichungstag der Anmeldung: **12.11.80 Patentblatt 80/23**

(45) Bekanntmachung des Hinweises auf die Patenterteilung: **30.09.81 Patentblatt 81/39**

(84) Benannte Vertragsstaaten: **CH DE FR GB LU SE**

(56) Entgegenhaltungen:
**FR - A - 1 319 594**
**FR - A - 2 160 611**

(73) Patentinhaber: **HOECHST AKTIENGESELLSCHAFT**
**D-6230 Frankfurt am Main 80 (DE)**

(72) Erfinder: **WEBER, Rolf-Ortwin**
**Erbensacker 18**
**6200 Wiesbaden-Naurod (DE)**
Erfinder: **PERREY, Klaus**
**Hermann-Kohl-Strasse 1**
**4018 Langenfeld (DE)**
Erfinder: **RECHENBERG, Wolfrad**
**Kirberger Strasse 39**
**D-6251 Kaltenholzhausen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 018 360 B1

N-(5-Methoxybenzofuran-2-ylcarbonyl)-N'-benzylpiperazin und Verfahren zu dessen Herstellung.

Es sind schon N,N'-disubstituierte cyclische Diamine und deren Säureadditionssalze sowie deren therapeutische Eigenschaften bekannt. So ist in der FR—A—2 160 611 die Verbindung

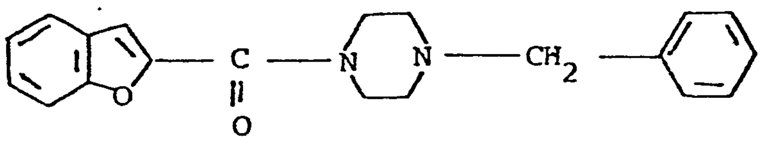

und deren Eignung als Psychotherapeutikum beschrieben. Bei dieser Verbindung handelt es sich um eine spezielle Ausführungsform von Verbindungen der allgemeinen Formel

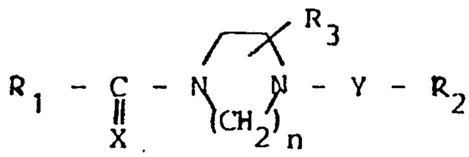

von denen es heißt, daß ihr Rest $R_1$ ein ein- oder mehrkerniger heterocyclischer Rest mit 4 bis 10 C-Atomen im cyclischen System sei, der als Heteroatom mindestens ein Sauerstoff-, Stickstoff- oder Schwefelatom enthalte und gegebenenfalls mindestens eine Doppelbindung sowie gegebenenfalls mindestens einfach durch mindestens einen Substituenten der Gruppe Halogenatome, Trifluoromethyl, Hydroxy, Alkoxy, Amino und Alkyl mit 1 bis 6 C-Atomen substituiert sei. Auch die Definitionen für X, Y, $R_2$ und $R_3$ können in verschiedener Weise variiert sein. In der Druckschrift heißt es dann weiter, daß die Alkoxygruppen nach einer bevorzugten Ausführungsform bis zu 3 Kohlenstoffatome enthalten. Zwar werden in der genannten Druckschrift nich weniger als 58 spezielle Verbindungen beschrieben, aber keine einzige Benzofuran Verbindung, in der ein Benzofuranrest mit Alkyl oder Alkoxy substituiert ist. Wohl wird jedoch der Grundkörper

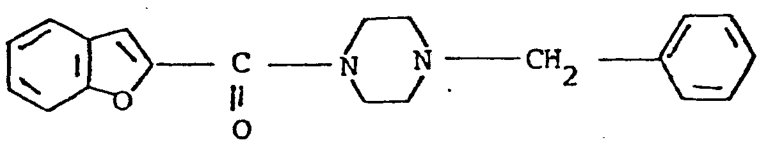

in dieser Druckschrift beschrieben.

Die in der FR—A—2 160 611 beschriebenen Substanzen haben sich zwar gut bewährt, es war jedoch erwünscht, die Stoffklasse der cyclischen Diamine dadurch auszubauen, daß man ausgewählte Substituenten gezielt in die 5-Position eines Benzofuransystems einführte, wobei Verbindungen mit einem gewissen Strukturbezug zu dem Neurotransmitter Serotonin erhalten wurden.

Die Erfindung betrifft nunmehr N-(5-methoxybenzofuran-2-ylcarbonyl)-N'-benzylpiperazin der Formel

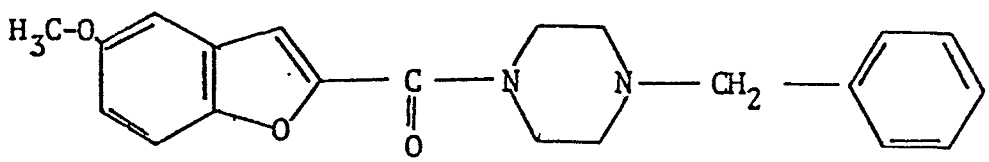

und dessen Säureadditionssalze mit einer physiologisch verträglichen Säure.

Im Rahmen der Erfindung liegt auch ein Verfahren zur Herstellung der obigen Verbindung, welches dadurch gekennzeichnet ist, daß in an sich bekannten Weise

a) N-Benzylpiperazin und 5-Methoxycumarilsäure
  - a1) bei erhöhter Temperatur ohne Verwendung von Kondensationsmitteln oder
  - a2) bei einer Temperatur von mindestens 15°C in Gegenwart von geeigneten Kondensationsmitteln oder

b) N-Benzylpiperazin mit einem aktivierten Derivat der 5-Methoxycumarilsäure, wie einem Anhydrid, Halogenid, Ester, Amid oder Azid, oder

c) N-(5-Methoxycumaroyl)-piperazin mit einem Benzylhalogenid -alkylsulfonat oder -arylsulfonat umgesetzt wird.

Bei Umsetzung a1) erzwingt man die Amidbildung unter Wasserabspaltung thermisch, indem die beiden Reaktionspartner vorzugsweise ohne Lösungsmittel auf Temperaturen von 130 — 280°C, bevorzugt auf über 150°C erhitzt werden. Diese Ausführungsform stellt eine besonders einfache und schnelle Verfahrensweise dar, mit der sich eine nahezu vollständige Umsetzung erzielen läßt. Zweckmäßig wird hierbei das Reaktionsgemisch so lange in dem oben angegebenen Temperaturbereich gehalten, bis die Abspaltung des Wassers, das vorteilhaft kontinuierlich aus dem Ansatz abdestilliert wird, beendet ist. Das Austragen des Reaktionswassers läßt sich durch das Hindurchleiten eines inerten, an der Umsetzung nicht teilnehmenden Gases, Wie Stickstoff oder eines Edelgases beschleunigen.

Wenn man die freie Säure und das freie Amin in Gegenwart von die Carbonsäureamidbildung fördernden Substanzen gemäß Verfahrensweise a2) umsetzt, arbeitet man beispielsweise im Temperaturbereich von 20 bis 150°C, vorzugsweise bei Temperaturen bis zum Siedepunkt des mitverwendeten Lösungsmittels, z.B. Tetrahydrofuran oder der weiter unten genannten Lösungsmittel. Als wasserabspaltende, die Carbonsäureamidbildung fördernde Substanzen kommen vor allem Carbodiimide, z.B. 1,3-Dicyclohexylcarbodiimid, 1 - Cyclohexyl - 3 - [2 - (4 - morpholinyl) - äthyl]-carbodiimid, 1 - (3 - Dimethylaminopropyl) - 3 - äthylcarbodiimid-hydrochlorid, 1,3 - Di - p - tolyl - carbodiimid, 1,3-Diisopropylcarbodiimid, aber auch Dialkylcyanamid mit 1 bis 4 C-Atomen im Alkylrest, z.B. Diäthylcyanamid, in Frage. Diese Dehydratisierungsmittel sowie das Diamin und die Säurekomponente werden zweckmäßig in äquimolaren Mengen eingesetzt. Dieses optimale Verhältnis kann zweilen auch über- oder unterschritten werden.

Bei Verfahrensvariante b) geht man von einem aktivierten Derivat der Carbonsäure aus. Besonders geeignet sind beispielsweise deren Halogenide, vorzugsweise das Chlorid oder Bromid, deren Ester von Alkoholen mit vorzugsweise 1 bis 4 C-Atomen oder von gegebenenfalls mit Nitro oder Chlor substituierten Phenolen; deren symmetrisches Anhydrid oder deren gemischte Anhydride, bevorzugt von Alkyl-, Phenylalkyl- oder Phenylkohlensäurehalbestern, wobei die Alkylgruppen jeweils 1 bis 4 C-Atome enthalten können; oder deren Amide, insbesondere von Azolen und Azinen, wie Imidazol, Triazol, Benztriazol bzw. s-Triazin, die durch Umsetzung äquimolarer Mengen der Carbonsäure und N,N'-Carbonyl-diimidazol, -triazol, -benztriazol bzw. -s-triazin bei Raumtemperatur in Tetrahydrofuran, Chloroform oder ähnlichen, gegenüber den Reaktionspartnern indifferenten Lösungsmitteln leicht herstellbar sind [(vgl. H.A. STAAB, Angew. Chem. *74,* 407 (1962)].

Bei Verwendung des Carbonsäurehalogenids und ebenso bei Umsetzung von N-(5-Methoxycumaroyl)-piperazin mit einem Alkylierungsmittel gemäß Verfahrenweise c) wird vorteilhaft in Gegenwart von Alkalicarbonaten, wie Natrium- oder Kaliumcarbonat, eines tertiären Amins, wie Pyridin, Picolin oder Triäthylamin bzw. mit einem molaren Überschuß des cyclischen Diamins, in der Regel in einem inerten Lösungsmittel, wie Benzol, Toluol, Xylol, bei einer Temperatur von 15 bis 50°C, gegebenenfalls bis zum Siedepunkt des Löungsmittels, gearbeitet. Eine bevorzugte Ausführungsform dieser Verfahrensweise besteht in der Umsetzung in Dimethylformamid. Dabei dient das Endprodukt gleichzeitig als Säurefänger und fällt als Hydrohalogenid an. Die bei dieser Ausführungsform primär gebildeten Salze können gegebenenfalls in an sich bekannter Weise, z.B. durch doppelte Umsetzung, in andere Säureadditionssalze übergeführt werden.

Ein besonderer Vorteil der Umsetzung in Dimethylformamid ist dessen erhebliche Reinigungswirkung. Auch aus diesem Grund ist dieses Verfahren vorzuziehen. Die Isolierung des Produkts kann auf übliche Weise, z.B. durch Ausfällen, erfolgen. Darüber hinaus ist die Reaktion vom Typ b), vorteilhaft die Umsetzung von N-Benzylpiperazin mit einem 5-Methoxycumarilsäurehalogenid, in Dimethylformamid besonders leicht technisch durchführbar; sie verläuft ohne störende Nebenreaktionen und liefert demzufolge hohe Ausbeute. Das Molverhältnis der Komponenten sollte dabei 1:1 gewählt werden; es sind jedoch auch andere Molverhältnisse, z.B. 3:1 bis 1:3, vorzugsweise 1,5:1 bis 1:1,5 möglich. Das benutzte Lösungsmittel kann wieder verwendet werden und die Reaktionskomponenten sind günstig verfügbar.

Für die Überführung der als Base anfallenden erfindungsgemäßen Verbindung der Formel I in physiologisch verträgliche Säureadditionssalze eignen sich beispielsweise Halogenwasserstoffsäuren, insbesondere Salzsäure, Schwefel-, Phosphor-, p-Toluolsulfon-, Methansulfon- und Cyclohexylamidosulfonsäure.

Die erfindungsgemäße Substanz zeichnet sich durch gute therapeutische Eigenschaften aus. So zeigt sie im Tetrabenazin-Katalepsie-Test einen wesentlich stärkeren Effekt als die handelsübliche Vergleichssubstanz Imipramin und darüber hinaus eine erheblich geringere Toxizität, so daß sich für sie ein wesentlich höherer therapeutischer Index ergibt. Die erfindungsgemäße Verbindung eignet sich daher als psychotherapeutisches Heilmittel, das weitgehend frei von unerwünschten Nebenwirkungen ist, durch welche die therapeutische Anwendung anderer Heilmittel eingeengt wird. Insbesondere zeigt die erfindungsgemäße Verbindung in thererapeutisch relevanten Dosen keine cardiotoxischen Wirkungen, keine zentrale Erregung, keine Sedation und keine Beeinflussung des vegetativen Nervensystems. Damit unterscheidet sie sich vorteilhaft von den vergleichbaren bekannten antidepressiv wirksamen Substanzen.

Die Stabilität der kristallinen Verbindung und ihrer Säureadditionssalze erlaubt die Herstellung von Arzneimittelzubereitungen, z.B. für orale, parenterale und rektale Verabreichung. Gegenstand der Erfindung ist somit auch die Verwendung der erfindungsgemäßen Substanz zur Herstellung von Arzneimitteln, insbesondere von Psychopharmaka, auf nicht-chemischem Wege. Ihre Dosierung kann z.B. 10 bis 400, vorzugsweise 50 bis 200 mg pro Tag betragen. In einer Dosierungseinheit können z.B. 10 bis 150, vorzugsweise 25 bis 100 mg enthalten sein.

Die Herstellung dieser Zubereitungen kann nach der üblichen Praxis unter Zumischung passender

und verträglicher Hilfsstoffe, wie Stärke, Milchzucker, Cellulosederivate, Stearinsäure oder ihrer Salze, Lösungsmittel, Lösungsvermittler, Zäpfchenmasse, Chloriden, Phosphaten und Carbonaten, z.B. Natriumbicarbonat, in Form von Pulvern, Tabletten, Dragees, Kapseln, Zäpfchen, Lösungen oder Suspensionen erfolgen. Aber auch die Verabreichung von Mikrokapseln ohne einen Zusatz ist möglich. Eine Ausführungsform der Erfindung besteht also in Arzneimitteln mit einem Gehalt an einer therapeutisch wirksamen Menge der erfindungsgemäßen Substanz.

## Beispiele

*1) a) 5-Methoxycumarilsäurechlorid*

576 g 5-Methoxycumarilsäure (3 mol) werden mit 625,5 Phosphorpentachlorid (3 mol) gut vermischt und mit 3 ml Phosphoroxytrichlorid versetzt. Während der exothermen Reaktion verflüssigt sich der Ansatz unter starker Salzsäuregasentwicklung. Anschließend wird an der Wasserstrahlpumpe das Phosphoroxytrichlorid abdestilliert und der Rückstand zweimal fraktioniert. Zur besseren Handhabung wird das heiße Destillat als Schmelze in dünnen Platten ausgegossen.

Ausbeute 593,7 g (94,0% der Theorie, Siedepunkt: 175°C (20mb), Schmelzpunkt 80—82°C.

*b) N-(5-Methoxybenzofuran-2-ylcarbonyl)-N'-benzylpiperazin-hydrochlorid*

Ein Gemisch aus 352 g N-Benzylpiperazin (2 mol) und 21 Dimethylformamid wird mit einer Lösung von 421 g 5-Methoxycumarilsäurechlorid (2 mol) in 600 ml Dimethylformamid versetzt. Nach einer exothermen Reaktion entsteht ein kristalliner Niederschlag, der beim Erhitzen wieder in Lösung geht. Man hält das Reaktionsgemisch 1 Stunde am Sieden und gießt es nach dem Abkühlen auf ca. 110°C unter starkem Rühren in 5 l Aceton. Der gebildete Kristallbrei wird auf Raumtemperatur abgekühlt. Zur vollständigen Ausfällung des Hydrochlorids wird die Suspension unter Rühren mit 37%iger wäßriger Salzsäure (ca. 100 ml) stark angesäuert. Man saugt das Festproduckt ab und wäscht so lange mit Aceton nach, bis das Filtrat farblos abläuft. Anschließend wird die in 95%iger Rohausbeute anfallende Verbindung nacheinander aus Dimethylformamid und Wasser umkristallisiert.

Ausbeute: 660 g (85% der Theorie), Schmelzbereich: 238 — 246°C unter Zersetzung, Base: 79°C (Diäthyläther).

2) Eine Lösung von 35,2 g Benzyl-piperazin (0,2 mol) in 250 ml Xylol wird mit 30,4 g fein gepulvertem wasserfreiem Kaliumcarbonat (0,22 mol) versetzt. Unter starken Rühren werden 42,1 g 5-Methoxycumarilsäurechlorid (0,2 mol), gelöst, in 200 ml Xylol, innerhalb von 5 Minuten bei Raumtemperatur zugefügt. Das Reaktionsgemisch wird 3 Stunden am Sieden unter Rühren am Rückfluß gehalten. Nach dem Abkühlen auf Raumtemperatur wird die Lösung vom Festprodukt abgesaugt und das Filtrat unter vermindertem Druck eingeengt. Der Eindampfrückstand wird in 300 ml Methanol aufgenommen und mit ätherischer Salzsäure versetzt. Der erhaltene Niederschlag wird mit Aceton und Äther gewaschen. Die Ausbeute an dem obengenannten Hydrochlorid beträgt 63,8 g entsprechend 82,5%. Zur weiteren Reinigung wird die Substanz aus Dimethylformamid und anschließend aus Wasser umkristallisiert.

3) Eine Lösung von 1053 g (5 mol) frisch destilliertem 5-Methoxycumarilsäurechlorid in 2 l Toluol wird der Lösung von 1760 g N-Benzylpiperazin (10 mol in 10 l Toluol) in einem Guß unter starkem Rühren zugesetzt, damit möglichst vor dem Ausfallen des Niederschlags ein homogenes Gemisch vorliegt. Es bildet sich schnell ein dicker Niederschlag, der das Reaktionsgemisch erstarren läßt. Es wird über einen Zeitraum von ca. 2 Stunden häufig umgeschüttelt bzw. umgerührt. Nach dem Abkühlen auf Raumtemperatur wird das Festprodukt auf einer Nutsche abgesaugt, mit Toluol und anschließend mit Aceton gewaschen. Die Festsubstanz besteht aus N-Benzylpiperazin-hydrochlorid, aus dem die Base zurückzugewinnen ist. Das Filtrat (Toluol-Aceton-Lösung) wird mit ätherischer Salzsäure versetzt. Das anfallende Festprodukt wird zuerst aus Dimethylformamid und dann aus Wasser umkristallisiert.

Ausbeute: 1702 g (88% der Theorie). Zuweilen kann die Ausbeute auch 92% betragen.

4) Eine Lösung von 21 g 5-Methoxycumarilsäurechlorid (0,1 mol) in 180 ml Pyridin wird mit einer Lösung von 17,6 g N-Benzylpiperazin (0,1 mol) in 70 ml Pyridin versetzt. Man läßt das Gemisch 30 Minuten lang unter Rückfluß sieden. Man kühlt ab, dampft das Lösungsmittel ab und kocht den Rückstand mit 200 ml Wasser auf. Nach erneutem Abkühlen nimmt man die Base in Methylenchlorid auf, wäscht die Methylenchloridlösung zweimal mit Wasser, trocknet über Natriumsulfat und entfernt das Methylenchlorid unter vermindertem Druck. Die so erhaltene Base kann nach üblichen Methoden in das Hydrochlorid überführt werden.

Ausbeute: über 80% der Theorie.

5) 22 g 5-Methoxycumarilsäureäthylester (0.1 mol) und 17,6 g N-Benzyl-piperazin (0.1 mol) werden in 100 ml Xylol unter Rückfluß zum Sieden erhitzt, bis die dünnschichtchromatographische Prüfung vollständige Umsetzung anzeigt. Man säuert das Reaktionsgemisch mit ätherischer Salzsäure an, saugt das Festproduckt ab und kristallisiert nacheinander aus Dimethylformamid und Wasser um. Man erhält das gewünschte 5-Methoxycumarilsäurepiperazid in Form einer farblosen kristallinen Substanz.

Schmelzpunkt: 238—246°C (Zersetzung).

6) 28,8 g 5-Methoxycumarilsäure (0,15 mol) und 26,4 g N-benzylpiperazin (0,15 mol) werden in 300 ml Tetrahydrofuran bis zur klaren Lösung erhitzt. Nach dem Abkühlen auf Raumtemperatur fügt man

eine Lösung von 30,9 g Dicyclohexylcarbodiimid (0,15 mol) hinzu, läßt über Nacht bei Raumtemperatur stehen und filtriert vom ausgefallenen Dicyclohexyl-harnstoff ab. Die Lösung wird eingeengt und der Rückstand mehrmals aus Äther umkristallisiert.

Man erhält die entsprechende Base vom Schmelzpunkt 79°C, die nach üblichen Methoden in das Hydrochlorid übergeführt werden kann.

7) 2,6 g 5-Methoxycumarilsäurepiperazid (0,01 mol) werden in 10 ml Xylol suspendiert und mit einer Lösung von 0,6 g Benzylchlorid (0,005 mol) in 5 ml Xylol versetzt. Man hält das Gemisch 1 Stunde auf Siedetemperatur, kühlt ab, trennt das Methoxycumarilsäurepiperazid-hydrochlorid ab und versetzt das Filtrat mit ätherischer Salzsäure. Der erhaltene Niederschlag wird isoliert und zuerst aus Dimethylformamid dann aus Wasser umkristallisiert.
Ausbeute: ca. 96,5%

Das isolierte 5-Methoxycumarilsäurepiperazidhydrochlorid kann nach Freisetzung der Base mit Natriumbicarbonatlösung bei weiteren Ansätzen verwendet werden.

8) Ein Gemisch von 57,5 g 5-Methoxycumarilsäure (0,3 mol) und 52,8 g N-Benzylpiperazin (0,3 mol) wird auf 250°C erhitzt, wobei ab etwa 170°C alles geschmolzen ist. Bei etwa 210°C beginnt die Wasserabscheidung, Man hält das Reaktionsgemisch so lange auf 250°C, bis die gewünschte Wassermenge (0,3 mol) abgeschieden ist. Der Rückstand wird in Äthanol gelöst, mit ätherischer Salzsäure versetzt und das anfallende Festprodukt mehrmals aus Dimethylformamid und zuletzt aus Wasser umkristallisiert.
Ausbeute: 65%.

*Pharmakologische Prüfung*

Die therapeutische Wirksamkeit der erfindungsgemäßen Substanz wurde im Tetrabenazin-Katalepsie-Test und im Reserpin-Antagonismus-Versuch an der Maus geprüft.

*Tetrabenazin-Katalepsie-Test*

Mäuse in Gruppen von je 10 Tieren erhalten 30 min. nach Applikation der Prüfsubstanz 15 mg/kg Tetrabenazin i.p. verabreicht. Das Auftreten eines kataleptischen Zustandes wird an einem zweistufigen Rundkorken beurteilt, an dem die Tiere so aufgesetzt werden, daß sie mit dem Kopf und den Vorderpfoten die untere, mit den Hinterpfoten die obere Stufe des Korkens berühren. Eine solche Position wird von einem normalen Tier umgehend korrigiert. Zur Beurteilung wird die Reaktion des Tieres jeweils 60 s beobachtet. Die Hemmung der Katatonie wird in Prozentwerten gegenüber der Kontrollgruppe errechnet.

Die in dieser anordnung erhaltenen Ergebnisse sowie die $LD_{50}$-Werte aus akuten Toxizitätsversuchen an der Maus sind in Tabellen 1 und 2 aufgeführt.

TABELLE 1

Tetrabenazin-Katalepsie-Test an der Maus Einfluβ auf die Katalepsiedauer

| Substanz | Dosis in mg/kg p.o. | Änderung der Katalepsiedauer gegenüber Kontrolle in % (min. post appl.) | | | n |
|---|---|---|---|---|---|
| | | 20 | 60 | 120 | |
| Erfindung | 10 | -62 ± 11 | -50 ± 5 | -62 ± 13 | 40 |
| | 20 | -62 ± 26 | -69 ± 3 | -57 ± 12 | 40 |
| | 40 | -81 ± 11 | -76 ± 15 | -82 ± 17 | 30 |
| Imipramin | 25 | -19 ± 19 | -38 ± 12 | -25 ± 17 | 30 |
| (Vergleich) | 50 | -23 ± 6 | -48 ± 9 | -34 ± 6 | 20 |

TABELLE 2

Toxizität an der Maus

| Substanz | n ( = Zahl der Tiere | $LD_{50}$ (Maus in mg/kg | |
|---|---|---|---|
| | | i.p. | p.o. |
| Erfindung | 5 | 548 | >4000 |
| Imipramin | 5 | 130 | 380 |

Aus Tabelle 1 ist ersichtlich, daß die erfindungsgemäße Verbindung im Tetrabenazin-Katalepsie-Test einen wesentlich stärkeren Effekt als die Vergleichsubstanz besitzt. Weiter wurde ermittelt, daß im Reserpin-Antagonismus-Versuch an der Maus die erfindungsgemäße Substanz wirkungsgleich mit dem Vergleichspräparat ist. Wie Tabelle 2 erkennen läßt, ist die verbindung gemäß der Erfindung bei oraler Applikation zudem noch mehr als 10 mal verträglicher als die Vergleichsverbindung so daß sich ein wesentlich höherer therapeutischer Index ergibt.

Die erfindungsgemäße Verbindung wurde außerdem auf ihren Einfluß auf den Coronardurchfluß und das Herzmechanogram am isolierten Meerschweinchenherzen nach Langendorff [Langendorff, O., Pflügers Arch. *61* (1895), 219] sowie auf ihre spasmolytische Wirkung am isolierten Meerschweinchendarm nach Magnus [Magnus, R., Pflügers Arch *102* (1904), 123] untersucht. Hierbei wurde die spasmolytische Wirkung jeweils gegenüber 0.2 $\mu$g/ml Histamin und $\mu$g/ml Bariumchlorid ermittelt. Die $ED_{50}$-Werte der erfindungsgemäßen Substanz und die Werte des zum Vergleich herangezogenen Handelsproduktes Imipramin aus diesen Versuchen sind in der Tabelle 3 zusammengestellt.

TABELLE 3

Wirkung am isolierten Meerschweinchenherzen und am isolierten Meerschweinchendarm

| Substanz | Dosis in $\mu$g | Wirkung auf das isolierte Meerschweinchenherz | | Spasmol. Wirkung als $ED_{50}$ in $\mu$g/ml gegenüber | |
|---|---|---|---|---|---|
| | | Änderung des Coronardurchflusses in % | Einfluß auf Kontraktionshöhe | Histamin | $BaCl_2$ |
| Erfindung | 50 | +39 | 0 | 5–10 | 5–10 |
| Imipramin | 10 | +19 /–18 | -25% | 0,001 | 0.1–1 |
| (Vergleich) | 20 | +28 /-29 | -25% | | |
| | 30 | +84 /-22 | -50% | | |

Wie Tabelle 3 zeigt, beeinflußt die erfindungsgemäße Verbindung die Kontraktionshöhe des isolierten Herzens nicht und führt zu einer Erweiterung der Coronargefäße. Im Gegensatz hierzu bewirkt die Vergleichssubstanz zwar zunächst eine kurzfristige Durchflußsteigerung, diese schlägt jedoch dann in eine Verengung um und hat damit eine Herzschädigung zur Folge, die in einer Verminderung der Herzkontraktion ihren Ausdruck findet.

Die spasmolytische Wirkung der erfindungsgemäßen Verbindung ist nur gering. Hieraus kann geschlossen werden, daß das periphere vegetative Nervensystem nicht oder nur geringfügig beeinflußt wird. Die Substanz kann dadurch nicht zu unerwüschten Nebenwirkungen, wie anticholinergen und Antihistamin-Effekten führen.

**Patentansprüche**

1. N-(5-Methoxybenzofuran-2-ylcarbonyl)-N'-benzylpiperazin der Formel

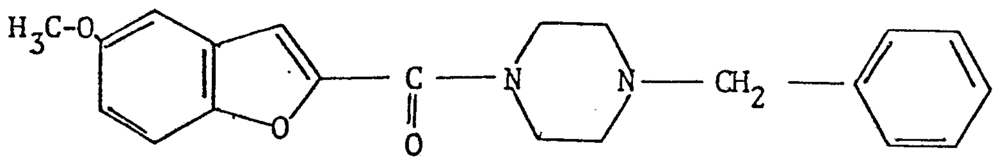

und dessen Säureadditionssalze mit einer physiologisch verträglichen Säure.

2. Verfahren zur Herstellung der Verbindung nach Anspruch 1, dadurch gekennzeichnet, daß in an sich bekannter Weise
a) N-Benzylpiperazin und 5-Methoxycumarilsäure
a1) bei erhöhter Temperatur ohne Verwendung von Kondensationsmitteln oder
a2) bei einer Temperatur von mindestens 15°C in Gegenwart von geeigneten Kondensationsmitteln oder
b) N-Benzylpiperazin mit einem aktivierten Derivat der 5-Methoxycumarilsäure oder
c) N-(5-Methoxycumaroyl)-piperazin mit einem Benzylhalogenid, -alkylsulfonat oder -arylsulfonat umgesetzt wird.

3. Verfahren nach Anspruch 2, dadurch gekennzeichnet, daß die Umsetzung a1) bei 130 bis 280°C durchgeführt wird.

4. Verfahren nach Anspruch 2, dadurch gekennzeichnet, daß die Umsetzung a2) in Gegenwart eines Carbodiimids oder eines Dialkylcyanamids erfolgt, wobei die Alkylgruppe jeweils 1 bis 4 C-Atome hat.

5. Verfahren nach Anspruch 2, dadurch gekennzeichnet, daß die Umsetzung b) mit einem Carbonsäurehalogenid und die Umsetzung c) jeweils unter Zusatz eines Alkalicarbonats oder eines tertiären Amins durchgeführt wird.

6. Verfahren nach Anspruch 2, dadurch gekennzeichnet, daß die Umsetzung b) und c) in Gegenwart von Dimethylformamid durchgeführt wird.

7. Verfahren nach Anspruch 2, 5 oder 6, dadurch gekennzeichnet, daß N-Benzylpiperazin mit 5-Methoxycumarilsäurehalogenid in Dimethylformamid umgesetzt wird.

8. Arzneimittel, gekennzeichnet durch einen Gehalt an einer therapeutisch wirksamen Menge der Verbindung nach Anspruch 1.

9. Arzneimittel nach Anspruch 8 zur Behandlung psychischer Erkrankungen.

10. Verwendung der Verbindung nach Anspruch 1 oder eines ihrer Säureadditionssalze zur Herstellung von Arzneimitteln auf nicht-chemischen Wege.

**Revendications**

1. N-(5-méthoxybenzofuranne-2-ylcarbonyl)-N'-benzyl-pipérazine de formule

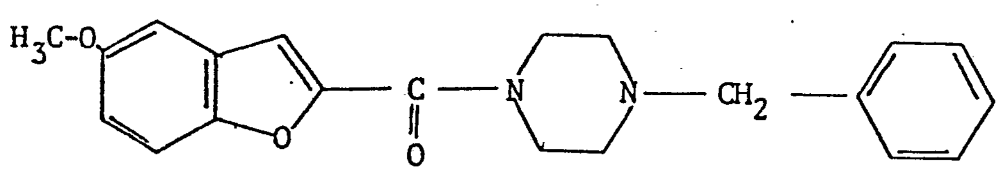

et ses sels d'addition avec des acides physiologiquement acceptables.

2. Procédé de préparation du composé selon la revendication 1, caractérisé en ce que, d'une manière connue en soi, on fait réagir:
a) la N-benzylpipérazine et l'acide 5-méthoxycoumarilique
a1) à température élevée sans utiliser d'agents de condensation, ou
a2) à une température d'au moins 15°C en présence d'agents de condensation appropriés, ou
b) la N benzylpipérazine avec un dérivé activé de l'acide 5-méthoxycoumarilique ou
c) la N-(5-méthoxycoumaroyl)-pipérazine avec un halogénure, un alkylsulfonate ou un arylsulfonate de benzyle.

3. Procédé selon la revendication 2, caractérisé en ce que la réaction a1) est conduite entre 130 et 280°C.

4. Procédé selon la revendication 2, caractérisé en ce que la réaction a2) est conduite en présence d'un carbodiimide ou d'un dialkylcyanamide, dont le groupe alkyle a de 1 à 4 atomes de carbone.

5. Procédé selon la revendication 2, caractérisé en ce que la réaction b) avec un halogénure d'acide carboxylique et la réaction c) sont conduites en ajoutant un carbonate alcalin ou une amine tertiaire.

6. Procédé selon la revendication 2, caractérisé en ce que les réactions b) et c) sont conduites en présence de diméthylformamide.

7. Procédé selon l'une quelconque des revendications 2, 5 ou 6, caractérisé en ce qu'on fait réagir la N-benzylpipérazine avec un halogénure de l'acide 5-méthoxycoumarilique dans le diméthylformamide.

8. Médicament caractérisé en ce qu'il contient une quantité thérapeutiquement active du composé selon la revendication 1.

9. Médicament selon la revendication 8 pour le traitement des maladies psychiques.

10. Utilisation du composé selon la revendication 1 ou d'un de ses sels d'addition avec des acides pour la préparation de médicaments par des voies non-chimiques.

**Claims**

1. N-(5-methoxybenzofuran-2-ylcarbonyl)-N'-benzylpiperazine of the formula

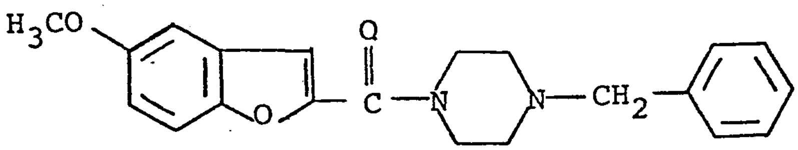

and its acid addition salts with a physiologically acceptable acid.

2. Process for the preparation of the compound of claim 1 characterized by reacting in a principally known manner

(a) N-benzylpiperazine and 5-methoxycumarilic acid

a1) at elevated temperature in the absence of a condensation agent or

a2) at a temperature of at least 15°C in the presence of suitable condensation agents; or

(b) N-benzylpiperazine with an activated derivative or 5-methoxy-cumarilic acid; or

(c) N-(5-methoxycumaroyl)-piperazine with a benzyl halide, -alkyl sulfonate or -aryl sulfonate.

3. Process of claim 2 wherein reaction a1) is performed at temperatures between 130 up to 280°C.

4. Process of claim 2 wherein reaction a2) is performed in the presence of a carbodiimide or of a dialkyl-cyanamide wherein each alkyl group has from 1 to 4 carbon atoms.

5. Process of claim 2 wherein reaction (b) with a carboxylic acid halide and reaction (c) each are performed in the presence of an alkali metal carbonate or a tertiary amine.

6. Process of claim 2 wherein reactions (b) and (c) are performed in the presence of dimethyl formamide.

7. Process of claims 2, 5 or 6 wherein N-benzyl-piperazine is reacted with 5-methoxycumarilic acid halide in dimethyl formamide.

8. Medicament characterized by a content of a therapeutically effective amount of the compound of claim 1.

9. Medicament of claim 8 for the treatment of psychic diseases.

10. Use of the compound of claim 1 or of one of its acid addition salts for the manufacture of medicaments by a non-chemical process.